# EUROPEAN PATENT SPECIFICATION

(11) **EP 3 139 964 B1**
(45) Date of publication and mention of the grant of the patent: **24.04.2019**
(21) Application number: 15723866.8
(22) Date of filing: 06.05.2015
(51) Int. Cl.: A61K 41/00, A61K 47/50, A61K 49/18, A61P 35/00, A61K 9/127, A61K 9/16

(54) **MAGNETOENZYMATIC CARRIER SYSTEM FOR IMAGING AND TARGETED DELIVERY AND RELEASE OF ACTIVE AGENTS**
MAGNETOENZYMATISCHES TRÄGERSYSTEM ZUR ABBILDUNG UND GEZIELTEN ABGABE UND FREISETZUNG VON WIRKSTOFFEN
SYSTÈME DE SUPPORT MAGNÉTO-ENZYMATIQUE POUR L'IMAGERIE ET L'ADMINISTRATION CIBLÉE ET LA LIBÉRATION D'AGENTS ACTIFS

(30) Priority: 06.05.2014 EP 14167196
(43) Date of publication of application: 15.03.2017
(73) Proprietor: Memorial Sloan Kettering Cancer Center, New York, NY 10065 (US)
(72) Inventor: GLÜET, Claus-Christian, 22587 Hamburg (DE); PENATE MEDINA, Tuula, 24159 Kiel (DE); PENATE MEDINA, Oula, 24159 Kiel (DE)
(74) Representative: Wallinger, Michael
(86) International application number: PCT/EP2015/059933
(87) International publication number: WO 2015/169843

(56) References cited:
- WO-A1-2011/112597
- WO-A1-2014/116866
- WO-A2-01/56546
- WO-A2-98/40049
- WO-A2-2011/147926
- WO-A2-2012/104275
- US-A1- 2003 211 045
- US-A1- 2009 004 258
- M Begoñ ET AL: "Different Effects of Enzyme-generated Ceramides and Diacylglycerols in Phospholipid Membrane Fusion and Leakage*", , 5 January 1996 (1996-01-05), XP055204867, Retrieved from the Internet: URL:http://www.jbc.org/content/271/43/2661 6.full.pdf
- STEFAAN JH SOENEN&#8203 ET AL: "Magnetoliposomes: versatile innovative nanocolloids for use in biotechnology and biomedicine", NANOMEDICINE, FUTURE MEDICINE LTD., LONDON, GB, vol. 4, no. 2, 1 February 2009 (2009-02-01), pages 177-191, XP008144157, ISSN: 1743-5889, DOI: 10.2217/17435889.4.2.177

## Description

### FIELD OF THE INVENTION

The present invention relates to a magnetoenzymatic carrier system in the form of a liposomal composition for imaging and targeted delivery and release of active agents as defined in the claims. Enzymatic degradation of phospholipids leads to the release of the carried active agents. The release rate can be amplified and modulated by applying an alternating magnetic field (AMF). In addition, the release rate can be monitored by imaging and regulated as desired. The present invention further relates to the use of the magnetoenzymatic carrier system in therapy and/or diagnostics, in particular to the use in therapy or diagnosis of illnesses typified by local apoptosis and necrosis like cancer and of inflammatory diseases like rheumatoid arthritis.

### BACKGROUND OF THE INVENTION

Liposomes and micelles are among the most common nanostructures used in clinical drug delivery applications. By preferentially enhancing localization of pharmaceutical activity in the organ or tissue of interest, their use has the potential to reduce the required systemic drug doses, thus minimizing the risks of adverse side effects while increasing treatment efficacy. Liposomes have been generated in multiple settings and in different formulations for clinical use and are generally well tolerated. Generally, liposomes are used for solubilization of drugs and prolonging their blood half-life. Liposomes also tend to accumulate at tumor tissues due to the enhanced permeation and retention (EPR) effect. Once the liposomes have accumulated at the target site, e.g., the cancer site, the active agents carried by the liposomes have to be released, preferably in an efficient and controllable manner, in order to obtain the desired treatment effects.

Thermosensitive liposomes designed to release drugs locally in response to hyperthemia are known for use in site-specific drug delivery. Near their gel to liquid crystalline phase transition temperature such thermosensitive liposomes release their encapsulated active agents more quickly and, thus, can achieve selective drug release. The drug release from thermosensitive liposomes by hyperthermia through the action of an alternating magnetic field (AMF) on paramagnetic particles is, for example, described in US 2009/0004258. Furthermore, drug release from thermosensitive liposomes using superparamagnetic particles embedded in the liposome membrane is known from WO 2011/147926, and drug release from liposomes using a paramagnetic entity is described in WO 2012/104275.

The drawback of thermosensitive liposomes is that they are very fragile to handle. In particular, thermosensitive liposomes suffer from a high leakiness in the blood of between about 5% to 20% per hour, which makes them poor vehicles for drug delivery. In addition, the known clinically used thermosensitive liposomes all have half-lifes in the blood of less than 30 minutes. Another problem is that the heating of the target tissue required for drug release also damages the surrounding tissue. Although intense efforts were directed towards limiting heat-induced tissue damages, it is still not possible to complete eliminate the risk of tissue damages.

Another possible strategy for selective drug delivery is based on the sphingomyelinase (SMase)-mediated release of liposome carried drugs. SMase is one of the key enzymes involved in cell stress response pathways. Elevated SMase activity is often associated with tumors, inflammation and necrosis, or it can be induced by cell stress factors like UV light, heat, oxidative stress, ionizing radiation, and chemotherapeutic agents (e.g., cisplatin, doxorubicin, gemcitabine, and etoposide). SMase converts sphingomyelin (SM) of SM containing liposomes (SM liposomes) to ceramide, and has been found to disrupts the liposome membrane and enable leakage of the contents of the liposomes in an animal model system (Oula Penate Medina, 2013).

However, in the treatment of human patients, the induction of SMase at the treatment site in a sufficient level to effect release of the active agents is not without problems. For example, if radiation is used to induce SMase activity, an undesirably high radiation dose has to be used in order to achieve a satisfactory release of the contents of SM liposomes.

The existing drug vehicles for site-specific drug delivery are therefore not quite satisfactory, and there is still room for improvements. In particular, while it is currently possible to image various disease states, such as inflammation or cancer, there has been only limited success to efficiently direct treatments specifically to the site that has been imaged. Furthermore, it is still impossible or difficult to specifically control or adjust the extent of drug release at the site of disease.

### OBJECT OF THE INVENTION

In view of the above, it is an object of the present invention to provide means and methods for targeted active agent delivery and release in an efficient and controllable manner.

### SUMMARY OF THE INVENTION

The above object is solved by a composition comprising liposomes (also referred to herein as "liposomal composition"), as defined in the claims, wherein the liposomes comprise:
(a) phospholipids, which may be of the same, single phospholipid type or a mixture of different (e.g., 2, 3, 4 or 5 different) phospholipids and which generally form the lipid bilayer (or "liposomal membrane" of the liposome), wherein the phospholipids comprise sphingomyelin (SM) (in which case the liposomes may be referred to herein as "sphingomyelin (SM) liposomes") and wherein the sphingomyelin content of the liposomes is in the range of 10 mol % to 45 mol %.
(b) magnetic nanoparticles, preferably magnetic nanoparticles comprising a metal, metal alloy, metal oxide, metal hydroxide, mixed metal oxide, mixed metal hydroxide, metal nitride, metal sulfide, mixed metal nitride, or mixed metal sulfide, wherein the metal is selected from the group consisting of chromium, manganese, iron, cobalt and nickel, especially from the group consisting of iron, cobalt or nickel, more preferably magnetic nanoparticles comprising iron, and most preferably magnetic nanoparticles comprising iron oxide (particularly Fe₃O₄) or a mixed oxide of iron and at least one additional metal;
   and wherein the liposomes further comprise one or both of components (c) and (d):
(c) an imaging agent and/or an imaging label, wherein the imaging agent and the imaging label are the same or, preferably, different and are preferably independently selected from the group consisting of radioactive isotopes, contrast agents (e.g., magnetic resonance contrast agents such as gadolinium complexes, magnetic particle imaging contrast agents, ultrasound contrast agents, photoacoustic contrast agents, X-ray contrast agents such as heavy metal or iodine containing compounds), fluorophores (e.g., a gadolinium chelate like indocyanine green (ICG)), and PET (positron emission tomography) labels,
(d) at least one active agent, wherein the at least one active agent is preferably a therapeutic agent and/or a nutritional agent (e.g., an antioxidant), particularly preferable a cytostatic agent; and
   wherein the liposomes optionally comprise
(e) additional compounds, including targeting molecules attached to the liposomal membrane.

In a preferred embodiment of the present invention, the liposomes comprise components (a), (b) and an imaging agent but no imaging label and no active agent(s). In another preferred embodiment, the liposomes comprise components (a), (b), and an imaging label but no imaging agent and no active agent(s). In another preferred embodiment, the liposomes comprise components (a), (b), an imaging agent and an imaging label but no active agent(s). In another preferred embodiment, the liposomes comprise components (a), (b), (d) and an imaging agent but no imaging label. In another preferred embodiment, the liposomes comprise components (a), (b), (d) and an imaging label but no imaging agent. In another preferred embodiment, the liposomes comprise components (a), (b) and (d) but no imaging agent and no imaging label. In a particularly preferred embodiment, the liposomes comprise components (a), (b), (d), and an imaging label and an imaging agent.

The liposomes used within the present invention are capable of releasing the imaging agent, if present (and comprised in the liposomes in a releasable form), and the at least one active agent, if present, into a target environment by the concomitant action of a phospholipase, which is able to degrade at least one phospholipid type of said phospholipids, and an alternating magnetic field (AMF).

As used herein, the term "imaging agent" refers to a molecule that can be detected using imaging techniques and that changes its signaling characteristics upon release of the liposome content at the target site (also referred to as "active" imaging agent). Thus, the imaging agent acts as indicator of release of the liposomal contents. Preferably, the imaging agent is located in the inside (i.e. the lumen) of the liposome but, however, it may also be embedded in the membrane or, less preferred, bound to the outside of the liposomal membrane. An "imaging label" within the meaning of the present invention refers to a molecule that can be detected using imaging techniques and that serves to monitor liposome delivery and localization (also referred to as "passive" imaging label). In other words, a liposome with an imaging label can be *in situ,* or *in vivo,* visualized. The imaging label may also act as reference signal against which the signal intensity of the imaging agent after release is compared. Preferably, the imaging label is bound (e. g., covalently attached to) the outside of the liposome membrane. However, it may also be embedded in the liposome membrane.

Within the context of the present invention, the term "diagnostic agents" means the imaging agent, the imaging label or a combination thereof. It is also contemplated within the scope of the present invention that the imaging agent can additionally act as an imaging label and *vice versa.* In this case only one molecule may be used for both the imaging agent and the imaging label (i.e. the imaging agent and the imaging label are the same chemical compounds).

The liposomal composition of the present invention enables the targeted delivery and release of one or more active agents to a target tissue and the efficient and specific release of the active agent at the target site, or into a target environment, by the concomitant action of an alternating magnetic field (AMF) and a phospholipase, which is able to degrade at least one phospholipid type of the liposome.

The invention is based on the surprising finding that the local enzymatic degradation induced active agent release of SM liposomes can be synergistically increased by the action of an alternating magnetic field (AMF) on magnetic nanoparticle incorporated in a SM liposome. This synergistic effect leads to the local pathophysiological effect of enzyme (SMase) being strong enough to trigger the release of active drugs and/or (active) imaging agents. As a result, the release is significantly stronger than that achieved by SMase alone. In particular, the desired release is achievable with the naturally occurring SMase enzyme concentration found normally in a given disease. In fact, the release is stronger than the SMase based release alone in SMase concentration enriched situations like after strong irradiation. In addition, it was found that the AMF effect is self-limiting in the sense that the heating of the magnetic nanoparticles is restricted to intact carriers with negligible heating once the membrane is degraded. Furthermore, the magnitude of the AMF strength required is more suitable (i.e. lower) for use in clinical settings than that proposed in the prior art approaches.

A suitable AMF for use herein is characterized by a magnetic flux density of about 60 mT or less, in particular 40 or 20 mT or less, preferably 10 mT or less, more preferably 5 mT or less and most preferably 3 mT or less. The lower limit of the AMF may be 0.1 mT, 0.3 mT, 0.5 mT or 1.0 mT. A preferred range is about 0.5 mT to 10 mT, in particular 1 mT to 7 mT, especially 2 mT to 5 mT. Furthermore, the field modulation frequency may be in the range of 50 kHz to 2000 kHz, typically in the range of 75 kHz to 1200 kHz, in particular in the range of 100 kHz to 1000 kHz. It is stressed that the indicated values correspond to a low to very low AMF level, which is much lower than that conventially used in the art.

The magnetoenzymatic carrier system of the present invention enables site specific regulation of liposomal active agent release at the target (e.g., tumor or infection) site. This strategy further opens the possibility to use cancer drugs, which already themselves have the ability to activate cellular SMase, thus leading to an activation loop. Furthermore, the liposomes used in the magnetoenzymatic carrier system of the present invention are initially not thermosensitive, which avoids the problematic leakiness of thermosensitive liposomes. In addition, the leakiness of the liposomes can not only be altered as a function of time, space and enzyme concentration (i.e. disease activity, reflected in cell stress which results in SMase to sensitize the liposomes or induced SMase activation) by means of adjustment of AMF strength but this process can be monitored (e.g., by fluorescent signals that change upon release of the (active) imaging agent) and thus controlled by imaging methods. This way treatment can be optimized with regard to location and intensity by using local AMF fields monitored by imaging methods.

In addition, the magnetoenzymatic carrier system described here may have imaging components that help to localize the carrier and provide information about the state of the carrier. Magnetic fields can be applied at the site of the accumulated carrier system and will result in the opening of the liposomes if a sufficient amount of SMase is present at the site. This leaves the non-activated carriers unaffected, i.e. carriers other than those close to the disease area will not become leaky and tissue surrounding the carrier system will not be directly heated.

Moreover, due to the fact that it is possible to detect the rate of release by monitoring changing fluorescent probe intensities and spectral shifts simultaneously, information can be gathered about location and severity of the inflammation or apoptotic and stress state of the cancer tissue. This information can be fed back to the AMF system which can be up- or down-regulated to adapt the release rate to the level deemed optimal. The level of control offered by the present invention through monitoring of endogenously produced release stimuli is not possible with current drug delivery systems.

In accordance with the present invention, the phospholipase is preferably a phosphatase that hydrolyzes sphingomyelin to ceramide, in particular sphingomyelinase (SMase), more preferably acid SMase (aSMase) or neutral SMase, and most preferably aSMase, and the phospholipid degraded by SMase is sphingomyelin (SM). SMase hydrolyzes SM to ceramide which is believed to cluster on the surface of the liposomal membrane, thereby destabilizing the liposomes and increasing their leakiness. The AMF results in a magnetic nanoparticle-mediated increase of the temperature, or more precisely of the brownian motion, of the liposomal membrane, which additionally destabilizes the liposomal membrane leading to increased leakiness.

The liposomes can have various chemical compositions as long as they can be used for the above-mentioned purpose. The preparation of liposomes is known in the art, including the preparation of liposomes that deliver active agents to a specific target site (site-specific delivery). As used herein, the term "targeted" is to be broadly construed and encompasses the use of antigen-antibody binding, ligand-receptor binding, and other chemical binding interactions, as well as non-chemical means. It also refers to the use of liposomes developed for targeting to a specific target within a subject or test sample using target components, moieties or groups, as known in the art. It further refers to passive targeting, e.g., by means of accumulation mediated by the EPR effect.

In accordance with the present invention, the magnetic nanoparticles have preferably a core diameter of between 2 nm and 10 nm, more preferably between 2 nm and 8 nm, and most preferably of about 5 nm. Preferably, the magnetic nanoparticles are integrated in the liposomal membrane.

The term "active agent", as used herein, is intended to refer to any compound sufficient to obtain the desired result and may include therapeutic agents, nutritional agents, and the like. Within the context of the present invention, the term "active agent" is interchangeably used with "active ingredient", and similar terms. The nature of the active agent is not particularly limited and may, for therapeutic use, include small organic molecules, enzymes, nucleic acids, antibodies, growth factors, proteins, peptides, carbohydrates, and nanoparticles. For nutritional use, the active agent may, for example, include microbes, prebiotics, vitamins, trace elements and antioxidants.

Preferably, the active agent is a cytostatic agent such as irinotecan, vinorelbine, gemcitabine, gefinitib, paclitaxel, taxotere, doxorubicin, cisplatin, carboplatin, BCNU, CCNU, DTIC, melphalan, cyclophosphamide, ara A, ara C, etoposide, vincristine, vinblastine, actinomycin D, 5-fluorouracil, methotrexate, herceptin, mitomycin C, or combinations thereof. Another preferred active agent is an antioxidant. Within the present invention, the active agent(s) is (are) preferably located in the inside of the liposome, i.e. the cavity formed by the liposomal membrane, but may also, or additionally, be incorporated in the liposomal membrane or bound to the surface of the liposome (i.e. the outside of the liposomal membrane).

The liposomal composition of the present invention are particularly advantageous in that they allow for the monitoring whether the targeting has been successful as a condition to initiate release of the payload (e.g., active agent(s)), and subsequently also control and, if needed, adjust the extent of release in a reliable and convenient manner. To obtain images of targeted tissues for monitoring delivery and at the same time delivering active agents to said targeted tissues, the above-mentioned imaging label may be bound to the outside of the liposomes or be embedded in the membrane. Furthermore, the release of an active agent carried in the inside of a liposome can be assessed by monitoring the change in signal originating from the active agent or, preferably, from the concomitant release of an imaging agent to the target environment (i.e. a target tissue).

Accordingly, the composition of the present invention allows delivery of an active agent to a target site and at the same time also allows (i) monitoring the distribution of the liposomes in the target tissue (e.g., by imaging labels bound or attached to the outside of the liposomal membrane) and/or (ii) assessing the release, including the extent of release, of the active agent by monitoring the change of a signal prior to and after release, such as a fluorescent signal, of an imaging agent carried by, preferably encapsulated in, the liposome. For example, an imaging agent may lead to a detectable fluorescence signal only after release from the liposome into the surrounding tissue, or the signal intensity may significantly change, or there may be changes in signal characteristics, e.g. in its spectrum, thereby allowing monitoring the extent of concomitant imaging agent release and, if required, to control the release by, for example, appropriately adjusting the AMF and/or the SMase activity. To this extent, the magnitude of the signal of the imaging label may serve as a reference to determine the relative amount of liposomes that have released their contents (or loads) (characterized by the signal changes of the imaging agent) relative to all liposomes in the target volume (characterized by the imaging label).

The composition of the present invention is therefore suitable for enzymatically and magnetically-controlled active agent release, and for use in monitoring the liposomes, wherein imaging labels or imaging agents are used for tracking, localization and quantification of the concentration of the liposomes and/or for monitoring the release of the active agents and/or for monitoring sphingomyelinase levels in the proximity of the liposomes.

The composition of the present invention may consist of only the liposomes describe herein, or comprise additional compounds, ingredients or substances. It is noted that the term "comprise", as used herein, is intended to encompass both the open-ended term "includes" and the closed term "consists of". Preferably, the composition of the present invention further includes a pharmaceutically acceptable carrier. As used herein, the term "pharmaceutically acceptable" refers to those compounds or substances which are, within the scope of sound medical judgment, suitable for contact with the tissues of mammals, especially humans. The term "carrier", as used herein, relates to a diluent, adjuvant, excipient or vehicle whereby the active ingredient is administered. Furthermore, one or more additional active substances may be included in the composition of the present invention that are co-administered with the liposomes. In addition, the composition may contain additional pharmaceutically acceptable substances, for example pharmaceutical acceptable excipients such as solubilizing agents, surfactants, tonicity modifiers and the like.

In another aspect, the present invention relates to a liposomal composition as described herein for use in therapy and/or diagnostics. As already mentioned, the composition of the present invention can particularly advantageously be simultaneously used for both therapeutic and diagnostic purposes or sequentially, i.e. first in diagnostic use and then in therapeutic or nutritional use, wherein the composition of the present invention to be used for diagnostic purposes is very similar or identical to the composition to be used for therapeutic or nutritional use except for the lack of incorporation of the active therapeutic or nutritional agent(s). By the use of one or several diagnostic agents (i.e. imaging agents/labels) the delivery, target accumulation and release pattern can be assessed for a given patient for the purpose of selecting the optimum candidate compound. Subsequently, the therapeutic or nutritional agents matching the optimum diagnostic agent may be administered to the patient. This type of application is commonly referred to as "companion diagnostics" and the invention described herein is particularly well suited for this kind of approach since it allows the incorporated compounds to be so well shielded that the delivery, accumulation, and release pattern of the diagnostic agent on the one hand and of the therapeutic or nutritional agent on the other hand are generally very similar, allowing for the accurate selection of the best therapeutic or nutritional agent among the candidate compounds tested.

The use in therapy typically includes the targeted delivery and release of an active agent to the treatment site, and the use in diagnostics typically includes the use of the composition as imaging means. The composition is generally used in an amount such that an effective amount of the active agent(s) is administered to the subject in need thereof. As used herein, the term "effective amount" refers to the amount of a compound sufficient to effect beneficial or desired results, in particular desired therapeutic results. An effective amount can be administered in one or more administrations, applications or dosages and is not intended to be limited to a particular formulation or administration route.

In a further aspect, the present invention relates to a liposomal composition as described herein for use in the treatment of cancer and/or for use in diagnosis of cancer, such as head and neck cancer. For example, SCC cell lines can be targeted via parenteral injection of SM liposomes containing encapsulated cisplatin and ICG. This cancer cell line is known to secrete aSMases and the accumulation can be imaged. The ceramide containing liposomes can be triggered to leak with local AMF pulse.

In yet another aspect, the present invention relates to a method of delivering and releasing an active agent to a target site, comprising the following steps
(a) providing a liposomal composition as described herein,
(b) administering the liposomal composition to deliver the liposomes contained in the liposomal composition to a target site; and
(c) applying energy to the liposomes and/or increasing SMase activity at the target site to effect release of the at least one active agent.

According to a preferred embodiment of the present invention, the method includes the additional step (step (d)) of monitoring the release of the active agent by measuring a signal of a first imaging agent prior to applying energy to the liposomes and/or increasing SMase activity at the target site and measuring a signal of the first imaging agent during and/or after applying energy to the liposomes and/or increasing SMase activity at the target site, wherein a change (i.e. increase or decrease) in the intensity of said signal indicates increased leakiness of the liposomes and release of the contents of the liposomes (i.e., of the at least one active agent and, usually, said first imaging agent). The magnitude of the signal of the imaging label may serve as a reference to determine the relative amount of liposomes that have released their contents (or labels) (characterized by the signal changes of the imaging agent) relative to all liposomes in the target volume (characterized by the imaging label).

In addition, the method preferably comprises the additional step (step (e)) of adjusting (or controlling) the extent of active agent release by adjusting the AMF and/or SMase activity at the target site. Moreover, the method may also include the additional step of monitoring the localization or accumulation of the liposomes at the target site by means of a second imaging agent, also referred to herein as "imaging label" or "passive imaging label". The second imaging agent is preferably bound or attached to the outer surface of the liposomal membrane. This additional step may be performed after step (b) but prior to step (c). It enables to determine whether the targeting has been successful and, if yes, to conduct step (c) to effect release of the active agent and, preferably, the first imaging agent.

The energy applied in step (c) generally results in a phase transition and/or change in the membrane structure and/or heating of the liposomal membrane, thereby effecting release of the at least one active agent (and any imaging agent optionally present in the inside of the liposome). Usually, the energy is applied by means of the magnetic nanoparticles under application of an AMF to the target site. The SMase activity in step (c) may be increased by administering SMase from the outside (exogenous SMase) or by applying means to induce endogenous SMase activity. Said means may, for example, include irradiation, exposure to UV light, application of heat, and administration of chemotherapeutic agents.

Preferred embodiments of the present invention are set forth in the appended dependent claims and in the following detailed description of the present invention.

### BRIEF DESCRIPTION OF THE DRAWINGS

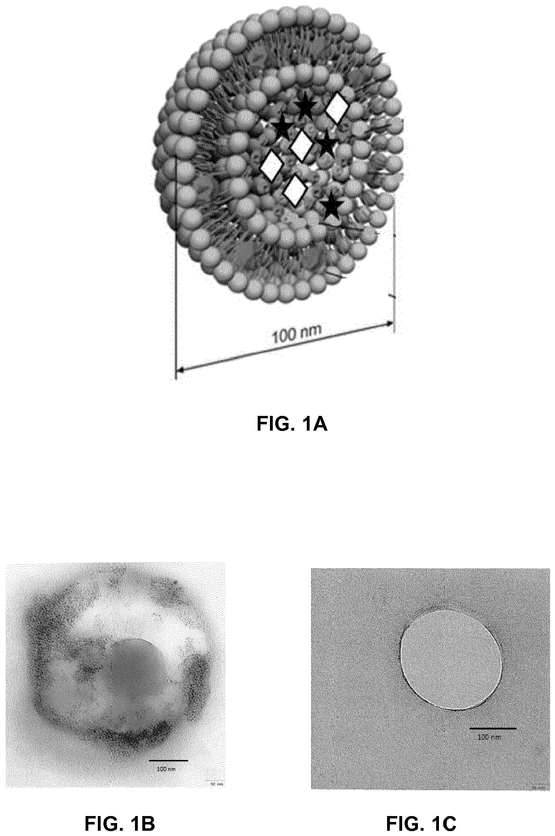

Fig. 1 schematically shows a magnetoenzymatic SM liposome according to the present invention. (A) Schematic illustration of SM liposomes containing Fe₃O₄-nanoparticles embedded in the lipid membrane (dark grey globular structures within the lipid bilayer liposomal membrane) and a payload of imaging molecules (*) and/or drug molecules (◇) inside the liposome. (B) TEM image of SM liposome (without membrane staining) with 5 nm Fe₃O₄ nanoparticles agglomerated in the lipid membrane. C. TEM image of SM liposome without Fe₃O₄ nanoparticles. The liposomal membrane was stained with uranyl acetate.

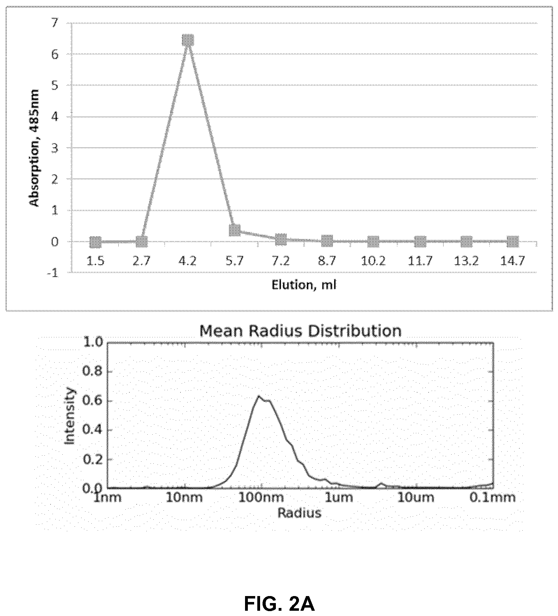

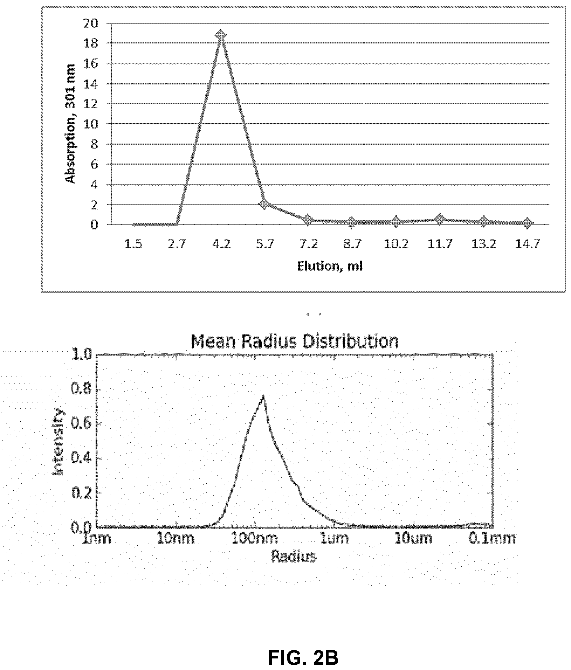

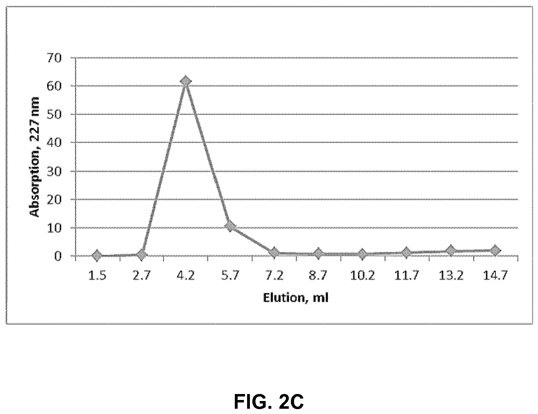

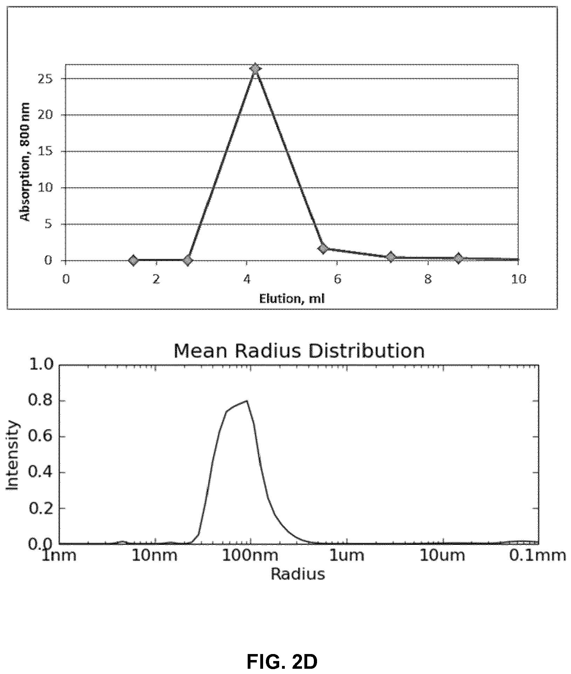

Fig. 2 shows validation of encapsulation of (treatment and/or imaging) agents and assessment of liposome size. Data for SM liposomes incorporating different types of anticancer drugs: (A) 0.5 mg/ml doxorubicin, (B) 4 mg/ml cisplatin, (C) 0.7 mg/ml paclitaxel; or for SM liposomes incorporating different types of imaging agents: (D) 0.5 mg/ml indocyanine green (ICG). Upper panels: typical elution curve from a PD-10 column for DSPC:SM:DOTAP:chol (20:30:20:30) liposomes with Fe₃O₄ nanoparticles (coated with catechol-PEG400 and carboxy group) on the membrane, containing the respective payload; fraction number three at volume 4.2 ml, containing the intact liposomes, was collected for downstream use. Lower panels: distribution of the mean radius of the SM liposomes as measured by dynamic light scattering analysis.

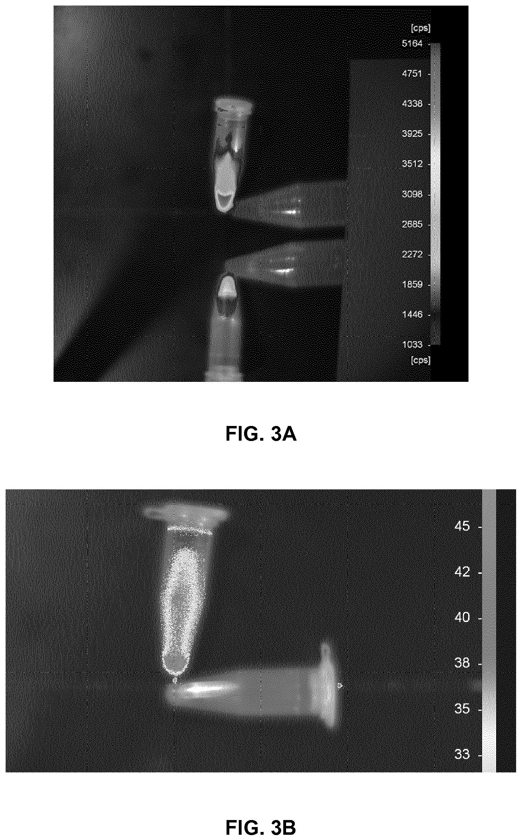

Fig. 3 shows the assessment of fluorescent signals of free and encapsulated agents. (A) Effect of breaking of the lipid membrane for doxorubicine in SM liposomes. Upper horizontal and vertical Eppendorf reaction vessels: SM Liposomes with doxorubicine and 5 nm Fe₃O₄ nanoparticles before (horizontal Eppendorf reaction vessel) and after (vertical Eppendorf reaction vessel) SM liposomes had been broken up with a 49 % EtOH solution. Lower horizontal and vertical Eppendorf reaction vessels: SM liposomes with doxorubicine (without Fe₃O₄ nanoparticles) before (horizontal Eppendorf reaction vessel) and after (vertical Eppendorf reaction vessel) SM liposomes had been broken up with a 49 % EtOH solution. (B) Effect of the breaking of the lipid membrane for ICG (with 5 nm Fe₃O₄ nanoparticles) in SM liposomes before (bottom right) and after (top left) treatment with 49 % EtOH solution to break the lipid membrane.

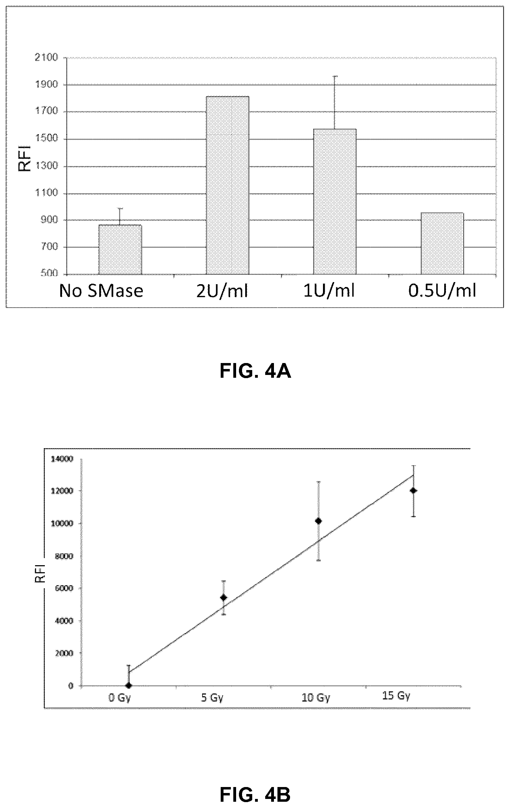

Fig. 4 shows the assessment of permeation of a SM liposome by SMase. (A) Liposome disruption *in vitro* by SMase. Lipid content 20 µmol, SM content 6 µmol. The relative fluorescence intensity (RFI) detected by FRET DNA hairpin assay is shown as a function of different levels of SMase exposure. (B) Liposome disruption *in vitro* by aSMase secretion by Human Aortic Endothelial Cells (HAoEC) induced by radiation stress with exposure levels ranging from 0 to 15 Gy. aSMase activity was measured using the sphingomyelinase activity assay kit from Cayman Chemical.

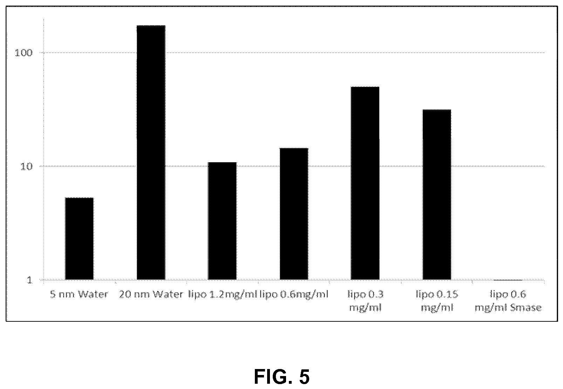

Fig. 5 shows the specific power absorption (SPA, W/g) of Fe₃O₄ nanoparticles in a liposome environment and free in water and exposed to an AMF of 23 mT at 828 kHz. Various concentrations of 5 nm Fe₃O₄ nanoparticles embedded in ICG loaded SM liposomes (1.2 mg/ml, 0.6 mg/ml, 0.3 mg/ml, and 0.15 mg/ml) were compared with 20 nm and 5 nm Fe₃O₄ nanoparticles free in water (the two leftmost columns). At optimum concentrations of 0.3 mg/ml, the embedded 5 nm nanoparticles demonstrate 10 times higher SPA values compared to 5 nm particles free in water. Upon addition of SMase in amounts sufficient to destabilize the membrane of the liposomes and release the iron oxides from the membranes, the SPA of the 5 nm particles was reduced below the detection limit.

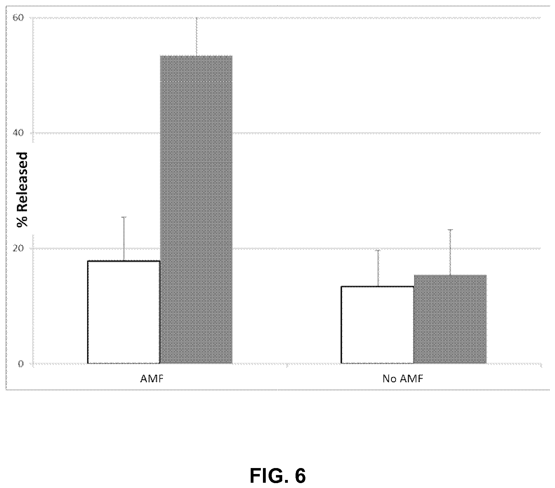

Fig. 6 shows the results obtained for SM liposomes in a leakage assay. The two bars on the left show the results for SM liposomes containing Fe₃O₄-nanoparticles (DSPC:SM:DOTAP:chol (20:30:20:30) mol/mol) with AMF exposure (1.5 mT, 100 kHz), and the two bars on the right show the result for SM liposomes without Fe₃O₄-nanoparticles (SM/DSPC/chol (30:40:30) mol/mol) and without AMF exposure. The white bars indicate no SMase exposure, and the dark bars indicate 0.8 U/ml SMase exposure. Exposure to both SMase and AFM resulted in a leakage rate that was about three times higher compared to only SMase or only AMF exposure.

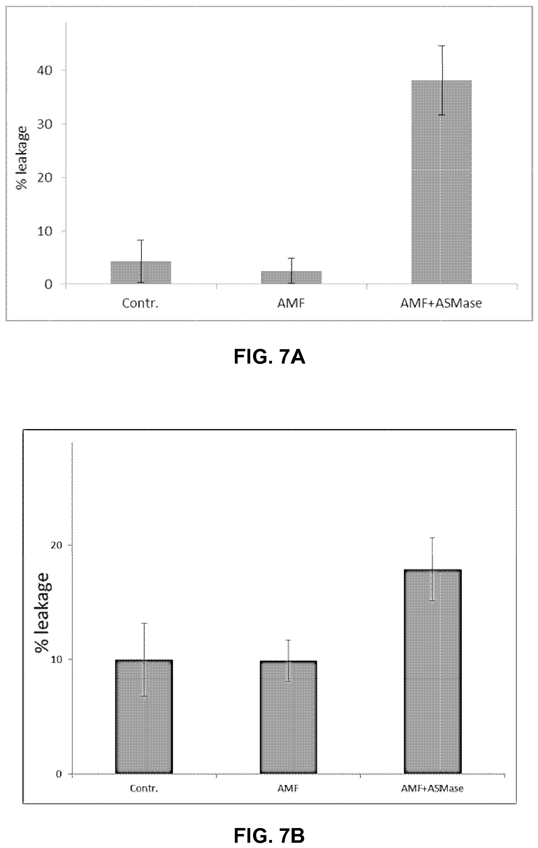

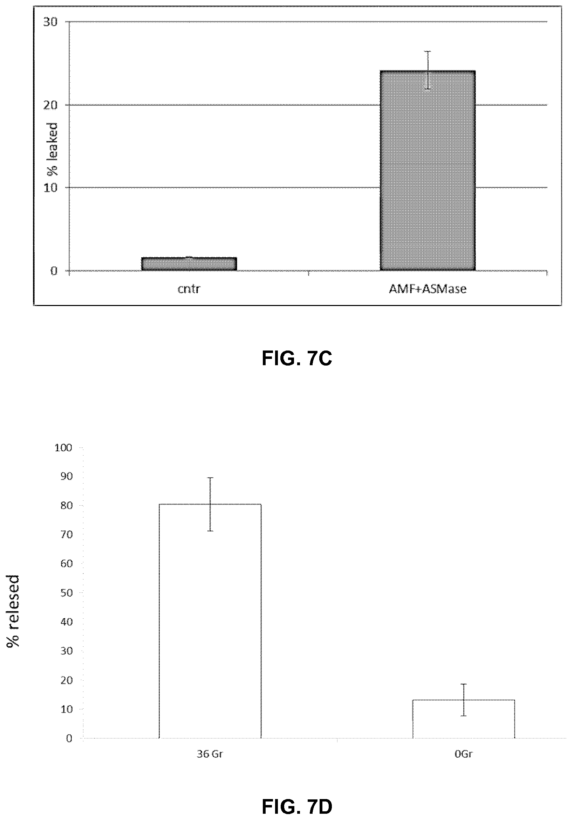

Fig. 7 shows the results obtained for different drug formulations in a liposome leakage assay. The SM liposomes were made of DSPC:SM:DOTAP:chol (20:30:20:30 mol/mol) with 5 nm Fe₃O₄ nanoparticles (0.6 mg/ml). (A) Doxorubicine (0.5 mg/ml) formulation. Left bar: without AMF and SMase exposure, middle bar: AMF only (5 mT, 229 kHz), right bar: AMF (5 mT, 229 kHz) after 30 min SMase (0.8 U/ml) pretreatment. (B) Cisplatin (4 mg/ml) formulation. Left bar: without AMF and SMase exposure, middle bar: AMF only (23 mT, 823 kHz), right bar: AMF (23 mT, 823 kHz) after 30 min SMase (0.8 U/ml) pretreatment. (C) Paclitaxel (0.7 mg/ml) formulation. Left bar: without AMF and SMase exposure, right bar: AMF (23 mT, 823 kHz) after 30 min SMase (0.8 U/ml) pre-treatment. (D) Leakage of doxorubicine (0.5 mg/ml) from SM liposomes after 1 h incubation with PC-3 cells exposed to aSMase induced by 36 Gy radiation (left) and without irradiation (right).

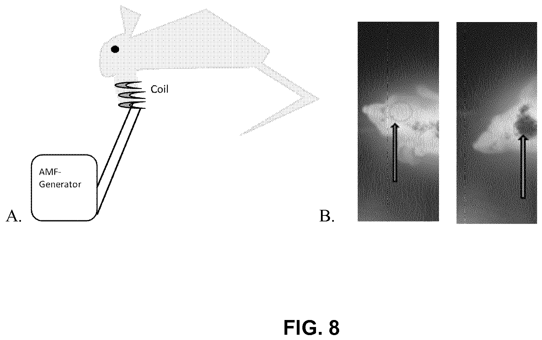

Fig. 8 illustrates the treatment of cancer in a mouse model using SM liposomes according to the present invention. (A) Schematic drawing of the experimental set up used in AMF experiments for the treatment of oral squamous cell carcinoma (SCC). The top of the coil is placed near the tumor and the tumor is exposed to AMF treatment. (B) Visualization of the release of an imaging marker from SM liposomes in an orthotopic mouse model of SCC4 oral squamous cell carcinoma. ICG encapsulated in 5 nm Fe₃O₄ nanoparticles containing SM liposomes does not yield a visible fluorescent signal prior to AMF treatment (left), but leads to a clearly visible fluorescent signal after release due to the AMF treatment (20 min, 1.5 mT, 100 kHz).

### DETAILED DESCRIPTION OF THE INVENTION

The present invention combines enzymatic action together with AMF triggered magnetic nanoparticle containing liposome release, resulting in a more specific and more selective release than can be achieved at physiological SMase concentrations found in various diseases and with AMF ranges suitable for use in clinical settings.

The liposome carriers of the invention feature a specific externally controllable release mechanism which allows the rupture of the liposome when being in correct place and when needed, leading to release of the payload. For this purpose two different mechanisms are combined: (a) degradation of sphingomyelin as a lipid constituent of the liposome by enzymatic action of SMase secreted by pathologic cells, e.g. in cancer and in inflammation, and (b) magnetic nanoparticles used for destabilizing the liposome membrane by the action of AMF, thereby inducing leakiness.

Furthermore, acid or neutral SMase secretion can be enhanced by localized irradiation, various stress stimulators like lipopolysaccharides, disruption of integrin signaling, UV-light, heat, oxidative stress and accumulation of Cu²⁺, participation of platelet-activating factor, chemotherapeutic agents (e.g., cisplatin, etoposide, gemcitabine, fenretinide, paclitaxel, rituximab, daunorubicin, Ara-C, and doxorubicin), or it can be activated via TNF- receptor superfamily members (Fas, CD40, DR5, and TNFα). These stress stimulators can be used to induce the increase of SMase at the site of illness which may release further compounds, which can again activate more aSMase and thus multiply the drug effect at the site of disease.

Within the context of the present invention, SMase from external sources may also be used. There are several parasites, bacterial strains and viruses that utilize SMase in their life cycle. This innovation allows the usage of these non-human sources of SMase to be detected and utilized for targeted release and imaging. This includes for example SMase secreting bacteria like *B*. *cereus* (Oda et., 2010), C. *pneumonia* (Penate Medina et al., 2014), and *S*. *aureus* (Hedström and Malmqvist, 1982), pathogens observed frequently in hospital environments, as contaminants in food and drinks and on material surfaces including, e.g., implants.

For the sphingomyelin (SM) liposomes according to the invention, commonly occurring SMs such as 16:0 SM, 16:0 SM (d18:1/16:0), 17:0 SM (d18:1/17:0), 18:0 SM (d18:1/18:0) may be used. The SM content of the liposomes is in the range of 10 to 45 mol%. In the context of the present invention, the SM content is in the range of 10 to 45 mol% or 15 to 40 mol%, e.g., 20 to 35 mol% or 25 to 35 mol%. The amount of SM as substrate for SMase has to be sufficient to induce creation of ceramide rich platforms that make the liposomes sensitive to the AMF treatment. A level of about 5% ceramide in the membrane has to be reached for ceramide-rich domains to form (Sot et al., 2006) and 10% of ceramide in the membrane for vesicle budding to happen (Nurminen et al., 2002).

Furthermore, other liposome forming lipids may be used such as those selected from the group consisting of phospholipids, tocopherols, sterols (e.g., cholesterol and derivatives thereof, ergosterol and derivatives thereof, lanosterol and derivatives thereof), glycoproteins, and mixtures thereof. The phospholipids may be selected from the group consisting of: (1) phosphatidylcholine (PC) (e.g., dioleoylphosphatidylcholine (DOPC), dilinoleoylphosphatidylcholine (DLPC), dimyristoylphosphatidylcholine (DMPC), dipalmitoylphophatidylcholine (DPPC), disaturated-phosphatidylcholine (DSPC), dioleoylphosphatidylcholine (DOPC), egg PC, hydrogenated soy PC); (2) phosphatidylglycerol (PG) (e.g., dimyristoylphosphatidylglycerol (DMPG), dipalmitoylphosphatidylglycerol (DPPG), distearoylphosphatidylglycerol (DSPG), egg PG); (3) phosphatidylethanolamine (e.g., dimethylphosphatidylethanolamine (DMPG), dipalmitoylphosphatidylethanolamine (DPPE), distearoylphosphatidylethanolamine (DSPG), dioleoylphosphatidyl ethanolamine (DOPE)); (4) phosphatidylserine (PS) (e.g., dioleoylphosphatidylserine (DOPS)); (5) phospatidic acid (e.g., dimyristoylphosphatidic acid (DMPA), dipalmitoylphosphatidic acid (DPPA), distearoylphosphatidic acid (DSPA)); (6) PEGylated derivatives of (1) to (5); (6) other lipids (e.g., N-(2,3-di-(9-(Z)-octadecenyloxy)-prop-1-yl-N,N,N-trimethylammonium chloride (DOTMA) and 1,2-bis(oleoyloxy)-3-(trimethylammonio)propane (DOTAP)), and combinations thereof.

Suitable liposomes for use herein comprise at least one sphingomyelin in an amount of, 10 to 45 mol%, 15 to 40 mol%, 20 to 35 mol% or 25 to 35 mol%) and at least one lipid selected from the group consisting of: phosphatidylcholine (PC) (e.g., DPPC, DSPC, DOPC and DMPC) in a total amount of about 10 to 85 mol% (e.g., 15 to 50 mol%, 15 to 40 mol% or 20 to 35 mol%), cholesterol (or derivatives thereof) in an amount of 5 to 40 mol% (e.g., 10 to 35 mol% or 20 to 30 mol%), phospatidylethanolamine (e.g., DMPE, DPPE, DSPE) in a total amount of 0.1 to 30 mol% (e.g., 0.1 to 20 mol%, or 1 to 10 mol%), phosphatidylserine (e.g., DOPS) in a total amount of 1 to 35% (e.g., 5 to 25% or 10 to 20 mol%), DOTAP and/or DOTMA in a total amount of 5 to 40 mol% (e.g., 5 to 30 mol%, 5 to 20 mol% or 10 to 25 mol%), and combinations thereof.

Preferably, the other liposome forming lipids (i.e. lipids besides the SM lipids as defined above) may be: cholesterol 10-30% mol%, phosphatidycholine (PC) 25-80 mol%, cationic lipids (DOTAP) 5-20 mol%, phosphatidylethanolamine (PE) 0.1-10 mol%, or phosphatidylserine (PS) 5-20 mol%. The preferred size of the liposomes is from 50 to 300 nm, more preferably from 80 to 250 nm, which allows sterile filtration prior to *in vivo* injection (using standard 0.22 micron sterile filters), but it may range up to 800 nm.

Accordingly, in the course of action of SMase on mixed SM/phosphatidylcholine membranes the generated ceramide forms microdomains (Nurminen et al., 2002). In terms of physical chemistry this represents an isothermal transition triggered by an enzymatic reaction, causing a shift in the lipid phase diagram from the fluid disordered phase into the two phase region, consisting of the fluid disordered and the solid ordered phases, the latter being enriched in ceramide. This isothermal transition will permanently alter the state of the liposome before and after enzymatic cleavage. Before SMase action the liposome is rigid and non-leaky, after SMase action the liposome is fragile and unstable. This instability of the lipid membrane allows to increase leakage by applying heat or by inducing increasing lateral movement on the membrane resulting in further destabilization of the membrane. These effects can be generated by AMF induction through beads agglomerated in the membrane and/or by external heat sources like ultrasound, laser or heating probes.

The liposomes, also referred to as primary carrier, according to the invention may carry medical agents, diagnostic agents, nutritional agent, a radiation sensitizer, a contrast agent, an enzyme, nucleic acid, an antibody, a growth factor, a protein, a peptide, carbohydrate, a targeting group or combinations of those.

For the magnetoenzymatically controlled active agent release by an AMF upon SMase action, the core size of the Fe₃O₄ nanoparticles may be in the range of 2 to 10 nm, preferably 5 nm. Also other magnetic, superparamagnetic, paramagnetic and ferromagnetic particles in the same size range can be used. Small nanoparticles incorporate spontaneously in the membrane. Carboxy coated 5 nm Fe₃O₄ nanoparticles associate spontaneously to the membrane (5 nm particles with cathechol-PEG-COOH group). When nanoparticles in the bloodstream have a size of 2 to 10 nm they can be cleared through the kidney. The iron oxide nanoparticles are therefore preferably 10 nm or smaller, but larger or equal to 2 nm. Kidney clearance is difficult or nearly impossible if the particle size is larger than 10 nm in mice. In humans the exact shape and size of the kidney threshold has not been established but it is thought to be comparable and vary with the age and health of the patient.

The magnetic field strength for drug release by AMF may be in the range of 0.3 mT to 60 mT, and the field modulation frequency in the range of 100 to 1000 kHz. The application of the AMF may be totally local and can be done after or during imaging, enabling total control of the process. On the other hand, the fact that SMase activity is only associated with severe disease conditions provides an insurance against agent release in false-positive areas of disease or off-target accumulation that will naturally arise from the uneven distribution of the liposomes in the body.

The enhanced sensitivity to AMF in the presence of SMase brings advantages for clinical application since it may permit the use of surface coils for generating the AMF. Small portable coils allow selective AMF induction at the sites of the tumor only. This spares the other parts of the body from drug release from the liposomes and from heating. It is known that energy dissipation, i.e. heat generation, is linearly dependent on the frequency, it increases with the second power of the magnetic field, and for surface coils it decreases with the third power of the distance. While this allows for more spatially selective exposure it may also limit the usage of small surface coils outside the body of the patients. Due to the synergistic effect of SMase and AMF the effective magnetic field used can be lower and this way the usability of the surface coils for initiating liposomal drug release is enhanced. While it also would be possible to place a patient inside a magnetic coil this requires higher magnetic fields which, due to medical risks, might be increase procedural complexity and may be contraindicated for some patients (e.g. with pacemakers) and medical personnel.

Unlike "magnetoenzyme" sensitive SM liposomes that - when exposed to AMF - will only open in the vicinity of pathologies expressing SMase, "thermosensitive" liposomes described in the state-of-the-art do not demonstrate such a selective release - all the liposomes in the magnetic field will become permeable once a certain level of energy is disposed. Moreover, in those systems the heating of the surrounding tissues continues even after liposomes have been broken as long as AMF exposure continues. Furthermore, thermosensitive liposomes, if equipped with imaging markers that change emission patterns upon release of the agent, will yield signals independent of the SMase state of the diseased target. Therefore, they lack the site specific SMase mediated information about disease activity reflected in the signal of the imaging marker released, a critical advantage in the assessment of disease status allowing to regulate and thus optimize the aggressiveness of therapy.

The combination of image control and disease mediated release rates provides the components required to realize a feedback loop system for adaptive and controlled therapy. Since AMF exposure allows to adjust release sensitivity to detectable levels of image signal modulation for varying levels of SMase expression this invention can also be used to follow the pathophysiological status of the cancer or the severity of inflammation by monitoring the release from SM liposomes caused by secreted SMase in the site of disease. The level of AMF required to detect a certain level of release of a marker would reflect SMase expression levels, e.g. associated with apoptosis, and thus can indicate both the effectiveness of the drug treatment and/or the severity of the illness.

This invention can also bring about improved methods for clearing the drug that did not reach the intended target pathology. For most cancer drugs 95.0 to 99.9 % of the injected drug never associate to the target tissue and target receptors (for an example involving doxorubicine see Gasselhuber et al., 2012). This off-target drug load causes adverse effects that limit the usability of the drug. The invention enables the encapsulated drug to circulate in the blood without interacting with off-target tissue. Clearance of off-target drug can be achieved in several different ways, e.g. by extracting the non-opened off-target iron oxide SM liposomes with a magnet from the blood (e.g. in a dialysis like setting).

The incorporation of iron nanoparticles in the liposomes was realized with the goal to improve the drug release efficacy and the better control of the drug release. An ideal nanoparticle would spontaneously associate to the membrane but dissociate after the liposome is made leaky because this would result in a self-limiting heating pattern, avoiding unnecessary heat generation that might lead to undesirable side effects. As nanoparticles for the magnetic action, the inventors used carboxyl functionalized Fe₃O₄ particles (catechol-PEG-COOH capped Fe₃O₄ particles, AC Diagnostics, Fayetteville, AR, USA). Small iron nanoparticles incorporated spontaneously to the lipid films. This may be due the opposite charges of the nanoparticles and the DOPA in the liposomes. On the other hand, phosphate that can be found in the phospholipid head groups is known to work as a stabilizer of the iron nanoparticles. It is also known that catechols have a tendency to leave the nanoparticles and this way allow the phosphate ions to interact with the iron core.

A schematic illustration of SM liposomes containing iron nanoparticles embedded in the lipid membrane stabilized by the phosphates of the lipids and containing payload (Fig 1A) shows that the payload active agent may be in the aqueous compartment inside the liposome. However, alternatively it also may be embedded in or attached to the membrane. Because isolated iron containing particles are too small to generate enough heat in aqueous solutions under AMF (Goya et al., 2008), they have to be clustered as depicted in figure 1B. This figure presents the TEM image of SM liposomes with 5 nm Fe₃O₄ nanoparticles. The agglomeration of the nanoparticles can be clearly seen. TEM imaging shows both clear association of the 5 nm nanoparticles on the membrane and large agglomerates in the membrane. The agglomerates also make the membrane to bulge. In the membrane of stained control liposomes without iron particles, smooth symmetrical contours are seen on the TEM image (Fig 1C).

The addition of Fe₃O₄ nanoparticles does not affect leakage. In the cationic liposomes, the Fe₃O₄ nanoparticles associate on the liposomes even though they are stabilized by catechol-PEG 400. This might be due to the dissociation of the catechol PEG coating from Fe₃O₄ nanoparticles and association of the Fe₃O₄ nanoparticles with phosphate groups of the phospho- and sphingolipids. Phosphate is a known stabilizer of iron nanoparticles and in this sense the iron particles could be stabilized on the liposomal water lipid interface where the charged head groups are available without additional stabilizers like catechols. Binding of iron nanoparticles to the membrane might also be strengthened by the opposite surface charges of the liposome membrane and iron-nanoparticle surface. Association of iron nanoparticles on the liposomal membrane is dependent of the state of the membrane and is reversible. Particles can dissociate from the membrane if the state of the membrane changes, for example when ceramide rich micro-domains are formed in the membrane.

PEG 400 may be used as solubilizing linker to the stabilizer catechol with a total MW of 760. This stabilizer linker complex is one order of magnitude smaller than the known efficient steric stabilizers. It is also known that catechols are more unstable than nitrocatechols and can dissociate from nanoparticles. These two facts allow the particle to interact with the surrounding phosphates from the phospholipids. The nanoparticles that have catechol DOPA and PEGs shorter that 1000 Da have a tendency to loosely associate with phospholipids when DOPA PEG can be depleted and this may help the nanoparticles to agglomerate at the membrane (Isa et al., 2010, and Goldmann et al., 2010). It is possible to consider the use of other stabilizers like oleic acid, silane, phosphate, and dopamine, since these stabilizers may be modified with a great variety of groups, as long as the particle associates and dissociates to/from the membrane.

Typically, the more stable dispersants such as nitro-DOPA-PEG5000 provide a dense, thin layer which is sufficiently thick to prevent nanoparticle agglomeration. PEGs from 5000 Da to 10 000 Da make the most stable nanoparticles that do not allow agglomeration and interaction of the iron nanoparticle with the lipid film. Reimhult et al. (2012) used Fe-particles of the roughly the same size as in the present invention but they used magnetic nanoparticles, which are stabilized using nitrocatechols with high-affinity palmitoyl-anchors to establish a thin but very dense hydrophobic coating, which can be incorporated into a membrane. Nitrocatechols are more stable and form less radicals than catechols (Amstad et al., 2009, and Amstad et al., 2010). However, this nitrocatechol bonding and anchoring is extremely stable and almost irreversible and makes the nanoparticle elimination from the body extremely difficult because the palmitoyl anchors can anchor the complex to any membrane.

Commonly occurring SMs were used, which can be in a bilayer membrane of liposomes such as 16:0 SM, 16:0 SM (d18:1/16:0), 17:0 SM (d18:1/17:0), 18:0 SM (d18:1/18:0). In the present invention the lipids used have been widely tested in clinical settings and thus are acceptable for clinical translation.

The SM liposomes containing drugs, ICG and iron nanoparticles were stable and the encapsulation efficiency was generally good. Doxorubicine (0.5 mg/ml) was encapsulated using a pH gradient and the encapsulation efficiency was high, as could be seen from the gel filtration elution curves. In a typical elution curve from a PD-10 column for DSPC:SM:DOTAP:chol (20:30:20:30) liposomes with Fe₃O₄ nanoparticles (coated with catechol-PEG400 and carboxy group), fraction number three contained the intact liposomes and was collected for further use (Fig 2A). Cisplatin (4 mg/ml) encapsulation was performed by combining the drug with the lipids when the lipids and cisplatin were hydrated. Fraction number three contained the intact liposomes and was collected for further use (Fig 2B) Paclitaxel (0.7 mg/ml) was encapsulated by dissolving paclitaxel in the lipid mix when in chloroform. Fraction number three contained the intact liposomes and was collected for further use (Fig 2C). ICG (0.5 mg/ml) was encapsulated with a similar strategy and added after the formation of the lipid film. Fraction number three contained the intact liposomes and was collected for further use (Fig 2D). Homogeneity and size distribution were checked by using dynamic light scattering (DLS).

To make the liposomes easier to break they can be created more fragile, more fluid like, by using lower amounts of cholesterol, and/or by using non saturated fatty acids in the lipid mixture. When these kinds of liposomes are used perhaps even smaller amounts of SMase, like found in infections, could be enough to break the liposomes but weaker liposomes have shorter storage half-life and are leaky in the blood stream. SM DSPC liposomes, on the other hand have large drug loading capacity, long shelf-life and robust circulation times. SM DSPC liposomes thus can be used to keep the liposomes always intact in physiological situations. Incorporation of iron nanoparticles makes them vulnerable only in case of exposure to magnetic fields that induce a phase transition. In this case, the initial sensitivity to low SMase concentrations may be reduced but better control of the release could be achieved.

Generally, the liposomes of or used in the present invention are not thermosensitive. The term "not thermosensitive", as used herein, preferably means that the liposomes are not sensitive (i.e. are stable, in particular in the sense that no leakage of, i.e., drugs or other active agents, occurs) in the typical range of body temperatures (e.g., 36-38°C or 36.5-37.5°C).

Liposomes that are composed of lipids that have Tₘ of 55 °C, DSPC of 41 °C sphingomyelin and of 4 °C cholesterol DOTAP are rigid and non-thermosensitive. They are also stable, with a shelf-life of several weeks. If not exposed to SMase (e.g., aSMase), phase transitions of liposomes with, e.g., sphingomyelin, cholesterol, phosphatidylcholine and DOTAP occur between 60-75°C, depending on the SM and helper lipids (i.e. all lipids except SM) concentrations. These such liposomes are not thermosensitive. Hence in the absence of SMase exposure (with or without AMF), no drug release is possible. For iron oxide containing variants, the liposomes of this rigidity do not leak either, even under AMF exposure. After SMase treatment, heat induced either directly or via ultrasound, or other thermal device would work as well as AMF, but applying heat to inner parts of the body in a controlled selective manner is challenging. The SM liposomes described here permit permeabilization even at low energy AMF (i.e. energies that do not increase the tissue temperature over 40 °C). Thus this avoids heat and other damage (e.g. by exposure to higher levels of ionizing radiation to induce higher levels of SMase).

The level and spectral distribution of ICG fluorescence is dependent on ICG concentration and the local environment. In vitro, ICG fluorescence is brightest at a concentration of 80 µg/ml and above that the fluorescence decreases rapidly when concentration increases (Mordon et al., 1998). In the experiments presented here the liposomal ICG concentration was 750 µg/ml, but the actual local concentration was several fold higher because the ICG was trapped in liposomes and possibly in the liposomal bilayer. This leads to an efficient quenching in cationic liposomes (Hua et al., 2012). The same quenching phenomenon applies to the doxorubicine liposomes: the doxorubicine fluorescence increases 10-fold when released from liposomes. Doxorubicine fluorescence can be used to monitor the release (Kheirolomoom et al., 2010).

To test if the fluorescence properties of ICG and doxorubicine could be used to monitor whether the agents are free or encapsulated in liposomes the following experiment was carried out. Doxorubicine (with or without iron oxide nanoparticles) or ICG were encapsulated in liposomes. After the liposome membrane was broken by 49% ethanol, an increase in the fluorescence signal was observed for both the free doxorubicine (Figure 3A) and the free ICG (Figure 3B). This effect can be explained by the p-p orbital stacking effect (Hua et al., 2012). The effect thus indeed can be exploited to monitor the release from liposomes and also to detect pathologic sites of high SMase expression and AMF exposure is to be used to bring imaging signals to levels detectable and monitorable. Such monitoring can be very informative when performed during surgery: intra operative imaging using near infrared markers such as ICG encapsulated in SM liposomes may be used to detect tumor tissue in order to verify successful surgery, i.e. complete removal of all tumor tissue. These optical signals can also be helpful as input for steering robot surgery devices.

In order to show that SMase treatment can release the molecules entrapped in the lumen of liposomes a liposomal hairpin test was carried out. A dose dependent increase of the fluorescent signal associated with SMase exposure was observed when hairpin DNA was liberated from liposomes. With this liposomal hairpin model it could be shown that there is leakage from SM containing liposomes when SMase concentration reaches 0.5 U/ml (Figure 4A). Figure 4B demonstrates that the level of aSMase required for permeation of SM liposomes can be produced by cells, in this case human aortic endothelial cells (HAoEC), exposed to levels of ionizing radiation as applied in clinical conditions like in cancer radiation therapy.

The effect of the applied AMF on the iron containing nanoparticles in liposomal or water environment and with different nanoparticle concentrations and sizes was studied in more detail by analyzing specific power absorption (SPA) values (W/g). Also the effect on SPA of exposure of the iron containing liposome system to SMase was studied. ICG containing SM liposomes with various 5 nm iron nanoparticle concentrations were investigated. For comparison, 20 nm and 5 nm iron nanoparticles were analyzed free in water (Figure 5). Three surprising observations were made.

First, a tenfold increase in the SPA values was measured when 5 nm particles were coupled to liposomes at optimal concentration levels ("lipo 0.3 mg/ml", Figure 5) vs. when they were free in water environment ("5 nm Water", Figure 5). When small 5 nm iron nanoparticles agglomerate in the lipid membrane they allow heating of the liposomes. In an optimum agglomerated state in the liposomal membrane 5 nm iron particles reach SPA values of about a third of the SPA values of 20 nm iron particles.

Second, the increase of the power absorption is dependent on the nanoparticle concentration in the liposomal environment. Still there is a limit: in a lipid membrane there is only space for limited amount of the agglomerated particles and this defines the upper limit for the SPA values. If more particles are added to the membrane they just are not optimally distributed to the membrane and that results in lower SPA values.

Third, the SPA effect for 5 nm iron containing nanoparticles in the liposomal membrane vanishes after exposure to SMase present in the solution ("lipo 0.6 mg/ml SMase" compared to "lipo 0.6 mg/ml" in Figure 5). This could be explained by the formation of ceramide rich microdomains that can destroy the agglomeration of the particles within the membrane. Particles released from the bilayer to the surrounding aqueous environment would lose the supernormal heating capability brought about by agglomeration.

It was also studied if there is synergy for drug release when using both AMF and SMase. Relatively low magnetic fields were used and enzyme amounts were chosen to simulate physiological conditions. The leakage of the ICG from ICG containing liposome with and without AMF and SMase treatment was measured. While enzyme treatment alone or the AMF exposure could induce only a limited leakage effect, an unexpected synergistic 3-fold increase of leakage of ICG from liposomes was induced by combined enzyme and magnetic field exposure (Figure 6).

Taken together, these various surprising observations reveal a very favorable drug release activation pattern for SM liposomes with nanoparticles of very small size (5 nm) that can agglomerate appropriately in the liposome membrane: a synergistic magnetoenzymatic effect permits reaching energy absorption levels high enough to cause leakage of the SM liposomes provided these are exposed to SMase. And once the liposomes have become leakier due to SMase exposure, the energy absorption is minimized, reducing any unnecessary further heat transfer to surrounding tissues after the payload has been released. This feature can be exploited to enhance the therapeutic window of AMF by either reducing potential side effects of heating or by permitting safe enhancement of energy absorption (adapting frequencies and magnetic field strength) for enhanced release of active drugs.

In order to confirm that release is not only feasible for imaging markers but also that the magnetoenzymatic carrier system can be used for drug release as well, additional experiments were performed. Doxorubicin, cisplatin, and paclitaxel were encapsulated and their release from the encapsulating SM liposomes by using the synergistic effect of combined SMase and AMF exposure was documented (Figure 7). The experimental setup was similar as for the liposomal leakage assays described above, exposing the carrier to SMase and/or AMF treatment at different magnetic field strengths and frequencies.

Figure 7A depicts results for the doxorubicine liposomal formulation for an AMF treatment at 5 mT with 229 kHz, while Figure 7B shows similar results for the cisplatin liposomal formulation with an AMF treatment at 23 mT with 823 kHz. Additional exposure to 0.8 U/ml of SMase led to an increase in the release rate, documenting that relatively low magnetic field strength may be sufficient, which may facilitate clinical application.

In Figure 7C results for yet another liposomal formulation are presented, in this case for paclitaxel with an AMF treatment at 23 mT with 823 kHz. In the release experiments of Figures 7A-C it has been shown that the drugs can be released by combined SMase and AMF exposure in a manner similar to the ICG release assays. The results vary for different drugs and are most impressive for the doxorubicine formulation, which showed a more than 10-fold increase in the case of additional SMase exposure, achieved at a relatively low magnetic field strength of 5 mT.

In the case of doxorubicine the leakage rate of liposomes when exposed to aSMase expressed by PC-3 cells by subjecting the cells to 36 Gy of ionizing radiation was investigated (Fig 7D). Extracellular expression of SMase and SMase induced ceramide are important reactions to cell stress and apoptosis, commonly observed in inflammation and cancer. Therefore the finding that SMase can be used to break specific types of liposomes, i.e. SM liposomes, makes it an interesting target to design drug delivery systems for endogenously triggered drug release. 2 U/ml SMase are enough to break SM liposomes but it this is a level that is rarely reached in typical pathophysiological settings. It has been reported that SMase activity of 0.5 U/ml can lead to 80% cell death in colon HT-29 cancer cells (Colell et al., 2002).

Fig. 6 shows that 30 min incubation with 0.8 U/ml SMase is not enough to release the content alone, but the additional exposure to AMF leads to a leakage rate of more than 50%. This supports the use of SM liposomes loaded with iron containing nanoparticles as drug delivery vehicles: once accumulated at sites of pathologic expression of SMase, AMF treatment will lead to the release of their payload, including drugs and/or imaging markers.

Using an experimental setup as depicted in Figure 8A it was tested whether the unquenching of ICG associated with the release from the liposome and observed *in vitro* (Figure 3) could be observed *in vivo.* ICG loaded Fe₃O₄ nanoparticle containing SM liposomes were injected into the tail vein of SCC4 orthotopic xenograft bearing mouse. The liposomes circulated for 15 min, then the animal was exposed to AMF (20 min at 1,5 mT, 100kHz). AMF treatment was directed to the tumor under the jaw, as depicted in Figure 8A. Near infrared imaging was performed in a NightOWL fluorescence imaging chamber 1 hour after the injection. As control, mice were injected with the same liposomal composition but not subjected to AMF treatment. The leaked ICG could be clearly seen in the tumor area under the chin of the AMF treated mouse (Fig 8B right) but not on the control mouse (Fig 8B left). This phenomenon with ICG quenching when encapsulated in liposomes is shown in Figure 3B.

The present invention will now be further illustrated by the following, examples.

### EXAMPLE 1

### Liposome formulations

Lipids were purchased from Avanti Polar Lipids and SMase (from B. *cereus)* from Sigma Aldrich.

### a) Doxorubicin loaded liposomes:

Liposomes were made using a lipid mixture consisting of a total of 20 µM lipids DPPC/Cholesterol/SM/DOTAP or DSPC/cholesterol/SM/DOTAP (20:30:30:20 mol%, respectively). In order to encapsulate doxorubicin into the liposomes a pH-gradient was used. Lipids stored in chloroform were pipetted to a round bottomed flask, dried under nitrogen and lyophilized for 24 h to remove trace amounts of chloroform. Lipids were allowed to hydrate for 30 min, at 50 °C in the case of DPPC and 60 °C for DSPC, in 1 ml of 250 mM (NH₄)₂SO₄, 150 mM NaCl, pH 4,1-buffer solution containing iron at a final concentration of 0.6 or 0.3 mg/ml after PD-10 purification (Fe₃O₄ particles coated with catechol-PEG400 and carboxy group).

The liposome solution was freeze-thawed 3 times and unilamellar liposomes were prepared with a needle tip sonicator (5x15 sec low energy on ice). The buffer was changed to PBS pH 6,5 by using a PD-10 column. Both doxorubicin (1,4 mM doxorubicin for 10 µM lipids) and liposomes were pre-heated to 50 °C if DPPC was used and to 60 °C if DSPC was used and then combined and incubated for 30 min at 50 °C or 60° C in a water bath. Liposomes were purified from the unbound compounds using a PD-10 column. Elution curves were established by using doxorubicin absorbance at 485 nm. Liposomes were controlled by liposome size measurements using dynamic light scattering (DLS).

### b) Cisplatin loaded liposomes:

Liposomes were made using a lipid mixture of DSPC/cholesterol/SM/DOTAP (20:30:30:20 mol%, respectively) consisting of a total of 20 µM of lipids. Lipids stored in chloroform were pipetted to a round bottomed flask, dried under nitrogen and lyophilized for 2 h to remove trace amounts of chloroform. Lipids were allowed to hydrate for 30 min in 60 °C buffered water solutions containing cisplatin (9 mM) and nanoparticle iron at a final concentration of 0.6 or 0.3 mg/ml after PD-10 purification (Fe₃O₄ particles coated with catechol-PEG400 and carboxy group). The liposome solution was freeze-thawed 10 times. Extrusion was performed 11 times through a 100 nm polycarbon membrane by using a small volume extruder, or unilamellar liposomes were prepared using a needle tip sonicator (4x15 sec low energy on ice). Liposomes were purified from the unbound compounds using a PD-10 column. Elution curves were obtained using cisplatin absorbance (at 301 nm in 0.1 M HCI). Liposomes were controlled by liposome size measurements using DLS.

### c) Paclitaxel loaded liposomes:

Liposomes were made using a lipid mixture of DSPC/cholesterol/SM/DOTAP (20:30:30:20 mol%, respectively) consisting of a total of 20 µM of lipids. Lipids stored in chloroform were pipetted to a round bottomed flask, dried under nitrogen and lyophilized for 2 h to remove trace amounts of chloroform. Paclitaxel was added to the lipids (0.7 to 1 mg) and traces of chloroform were lyophilized overnight. Lipids and paclitaxel were allowed to hydrate for 30 min at 60 °C in buffered water solutions containing Fe₃O₄ nanoparticles (0.6 mg/ml or 0.3 mg/ml final concentration). Liposome solutions were freeze-thawed 7-10 times. Extrusion was performed 11 times through a 100 nm polycarbon membrane using a small volume extruder, or unilamellar liposomes were prepared using a needle tip sonicator (5x15 sec low energy on ice). Liposomes were purified from the unbound compounds using a PD-10 column. Elution curves were obtained using paclitaxel absorbance (227 nm). Liposomes were controlled by liposome size measurements using DLS.

### d) Indocyanine green (ICG) loaded liposomes:

Liposomes were made using a lipid mixture consisting of a total of 20 µM of lipids (DSPC/Cholesterol/SM/DOTAP (20:30:30:20) in mol/mol ratios). Lipids stored in chloroform were pipetted to a round bottomed flask, dried under nitrogen and lyophilized for 2 h to remove trace amounts of chloroform. Lipids were allowed to hydrate for 30 min at 60 °C in buffered water solutions containing ICG (0.5 mg/ml) and Fe₃O₄ nanoparticles in final concentration 0.6 or 0.3 mg/ml after PD-10 purification (Fe₃O₄ particles coated with catechol-PEG400 and carboxy group). Extrusion was performed 11 times through a 100 nm polycarbon membrane using a small volume extruder, or unilamellar liposomes were prepared using a needle tip sonicator (3x15 sec low energy on ice). Liposomes were purified from the unbound compounds using a PD-10 column. Elution curves were obtained using ICG absorbance at 800 nm. Liposomes were controlled by liposome size measurements using DLS.

### EXAMPLE 2

### AMF treatment

The inventors used a coil with a magnetic field strength of 1,5 mT for *in vivo* experiments and the same 1,5 mT coil as well as a device with variable field strength ranging from 5-23 mT for *in vitro* studies.

AMF treatments were performed for 20 min (100 kHz, 15V, 1,5 mT) for each sample containing 300-500 µl liposomes. If a sample was pretreated, SMase 0.4 U were added (final activity 0.77 U/ml) 30 minutes before applying the AMF treatment. After treatment, samples were inserted into a snake skin dialysis bag and dialyzed for 24 h against PBS, or PBS pH 6,5 in order to analyze the amount of free doxorubicin, cisplatin, paclitaxel or free ICG. After 24 h incubation both solutions inside and outside of the bag were analyzed by measuring absorbance of doxorubicin, cisplatin, paclitaxel or ICG and the leakage rate was calculated.

AMF was also generated using instrumentation from nanoScale Biomagnetics (nB, Zaragoza, Spain). In this case, fields between 5-23 mT and 229-823 kHz were used. Different concentrations (0.15 to 1,2 mg/ml of Fe₃O₄ nanoparticles per 13 µmol/ml lipids in PBS) and also different sizes (5 to 20 nm) of Fe₃O₄ nanoparticles were incubated for 10 min in 5 - 23 mT in 750 µl, to study both leakage of the liposomes and magnetic hyperthermia, as well as to analyze specific power absorption values.

### EXAMPLE 3

### aSMase detection

aSMase detection was performed with the aSMase detection kit by Cayman Chemicals (USA) according to the instructions of the manufacturer. aSMase levels were measured 10 minutes after irradiation with 0 to 20 Gy gamma radiation.

### EXAMPLE 4

### In vivo imaging

*In vivo* imaging experiments were done with athymic nude mice bearing orthotopic SCC-4 carcinoma in the lower jaw region. Animals were anesthetized with Ketamine (110 mg/kg bw)/Xylazine (16 mg/kg bw) during handling and imaging.

After purification, liposomes containing ICG fluorophores and Fe₃O₄ nanoparticles in a volume of 150 µl were administered by tail vein injection. The mice were imaged with a Berthold NightOWL camera (Berthold Technologies, Bad Wildbad). Image analysis was performed with indigo software. Mice were also imaged with a fluorescent tomograph (FMT 2500, Perkin Elmer, Inc., Waltham, MA, USA) and under a surgical microscope (Moller-Wedel, Wedel, Germany).

Mice were positioned so that the tumor was inside the magnetic coil of the AMF apparatus. AMF (1,5 mT and 100 kHz) was applied to the tumor for 20 min. Mice were imaged after one hour together with the control mice that had gotten the same liposomal injection but had not been submitted to AMF. A clear increase in the ICG signal due to leaked ICG showed on the tumor area under the chin of the mouse.

### REFERENCES

Amstad et al., Ultrastable iron oxide nanoparticle colloidal suspensions using dispersants with catechol-derived anchor group. Nano Lett., 2009, 9, 4042-8.
Amstad et al., Influence of electronegative substituents on the binding affinity of catechol-derived anchors to Fe3O4 nanoparticles. J.Phys. Chem., 2011, 115, 683-91.
Colell et al., Divergent role of ceramide generated by exogenous sphingomyelinases on NF-κB activation and apoptosis in human colon HT-29 cells. FEBS Lett., 2002, 28, 15-20.
Gasselhuber et al., Comparison of conventional chemotherapy, stealth liposomes and temperature-sensitive liposomes in a mathematical model, PLoS ONE, 2012, 7, e47453. doi:10.1371/.
Goldmann et al., Biomimetic mussel adhesive inspired clickable anchors applied to the functionalization of fe(3) o(4) nanoparticles. Macromolecular Rapid Communications, 2010, 15, 1608-15.
Goya et al., Magnetic hyperthermia with Fe3O4 nanoparticles: the influence of particle size on energy absorption. IEEE Transactions on Magnetics, 2008, 44, 4444-7.
Hannun et al., Enzymes of sphingolipid metabolism: from modular to integrative signaling. Biochemistry, 2001. 40, 4893-903.
Hedström and Malmqvist. Sphingomyelinase activity of Staphylococcus aureus strains from recurrent furunculosis and other infections. Acta Pathol Microbiol Immunol Scand B., 1982, 90(3), 217-20.
Holopainen et al., Dimyristoylphosphatidylcholine/C16:0-ceramide binary liposomes studied by differential scanning calorimetry and wide- and small-angle x-ray scattering. Biophys. J., 2000, 78, 2459-69.
Hua et al., In vivo imaging of choroidal angiogenesis using fluorescence-labeled cationic liposomes. Mol. Vis., 2012, 18, 1045-54.
Isa et al., Self-assembly of iron oxide-poly(ethylene glycol) core-shell nanoparticles at liquid-liquid interfaces. CHIMIA, 2010, 64, 145-9.
Jaffrezou et al., Daunorubicin-induced apoptosis: triggering of ceramide generation through sphingomyelin hydrolysis. EMBO J., 1996, 15, 2417-24.
Kheirolomoom et al., Copper-doxirubicin as a nanoparticle cargo retains efficacy with minimal toxicity. Mol Pharm., 2010, 7, 1948-58.
Mordon et al., Indocyanine green: physicochemical factors affecting its fluorescence in vivo. Microvascular Research, 1998, 55, 146-52.
Nurminen et al., Observation of topical catalysis by sphingomyelinase coupled to microspheres. JACS, 2002,124, 12129-34.
Oda et al. Hemolysis induced by Bacillus cereus sphingomyelinase. BBA, 2010, 1798, 1073-80.
Penate Medina, Enzymatic degradation induced drug release and imaging of sphingomyelin liposomes, COST TD1004 Action, Theranostics Imaging and Therapy: An Action to Develop Novel Nanosized Systems for Imaging-Guided Drug Delivery, 2013, 08, 17).
Penate Medina et al., Identification of sphingomyelinase on the surface of Chlamydia pneumonia: possible role in the entry into its host cells. Interdisciplinary Perspectives on Infectious Diseases, 2014, Article ID 412827.
Reimhult et al., Nanoparticle actuated hollow drug delivery vehicles. Nanomedicine (Lond), 2012,7, 145-64.
Smith and Schuchman. The unexpected role of acidic sphingomyelinase in cell death and the pathophysiology of common diseases. The FASEB Journal, 2008, 22, 3419-31.
Sot et al., Detergent-resistant ceramide-enriched domains in sphingomyelin/ceramide bilayers. Biophys. J., 2006, 90, 903-14.
Stancevic and Kolesnick. Ceramide-rich platforms in transmembrane signaling. FEBS Letters, 2010, 584, 1728-40.
Strum et al., 1-P-D-habinofuranosylcytosine stimulates ceramide and diglyceride formation in HL-60 cells. J.Biol.Chem., 1994, 269, 15493-7.

## Claims

1. A composition comprising liposomes, the liposomes comprising:
(a) phospholipids, the phospholipids comprising sphingomyelin (SM),
(b) magnetic nanoparticles, and one or both of
(c) an imaging agent and/or an imaging label and
(d) at least one active agent,
wherein the sphingomyelin content of the liposomes is in the range of 10 mol% to 45 mol%, and
wherein the liposomes are capable of releasing an imaging agent and/or an active agent into a target environment by the concomitant action of sphingomyelinase (SMase) and an alternating magnetic field (AMF).

2. The composition of claim 1, wherein the magnetic nanoparticles have a core diameter of between 2 nm and 10 nm.

3. The composition of claim 1 or 2, wherein the magnetic nanoparticles comprise a metal, metal alloy, metal oxide, metal hydroxide, mixed metal oxide, mixed metal hydroxide, metal nitride, metal sulfide, mixed metal nitride, or mixed metal sulfide, wherein the metal is selected from the group consisting of chrome, manganese, iron, cobalt and nickel.

4. The composition of any one of claims 1 to 3, wherein the liposomes further comprise at least one imaging agent, including radioactive isotopes, contrast agents, fluorophores, and PET labels.

5. The composition of any one of claims 1 to 4, wherein the active agent is a cytostatic agent.

6. The composition of any one of claims 1 to 5, wherein the liposomes comprise a at least one therapeutic agent, at least one imaging agent, and an imaging label.

7. A composition according to any one of claims 1 to 6 for use in therapy and/or diagnostics.

8. The composition for use according to claim 7, wherein the use in therapy includes the use of the composition for the targeted delivery and release of an active agent to a target site, and the use in diagnostics includes the use of the composition as imaging means.

9. A composition according to any one of claims 1 to 6 for use in the treatment of cancer and/or for use in diagnosis of cancer.

10. A composition for use in a method of delivering and releasing an active agent to a target site, comprising the following steps:
(a) providing a composition according to any one of claims 1 to 6,
(b) administering the composition to deliver the liposomes contained in the composition to a target site; and
(c) applying energy to the liposomes and/or increasing SMase activity at the target site to effect release of the at least one active agent.

11. A composition for use according to claim 10, wherein the step of applying energy to the liposomes results in a phase transition and/or a change in the liposomal membrane structure and/or heating of the liposomes to effect release of the at least one active agent.

12. A composition for use according to claim 10 or 11, wherein in step (c) the energy is applied by applying an alternating magnetic field to the target site.

13. A composition for use according to any one of claims 10 to 12, wherein the SMase activity is increased by administering exogenous SMase or by applying means to induce endogenous SMase activity so as to effect release of the at least one active agent.

14. A composition for use according to claim 13, wherein the means to induce endogenous SMase activity include irradiation, exposure to UV light, application of heat, and administration of chemotherapeutic agents or cell stress inducers.

## Patentansprüche

1. Zusammensetzung, enthaltend Liposomen, wobei die Liposomen umfassen:
(a) Phospholipide, wobei die Phospholipide Sphingomyelin (SM) umfassen,
(b) magnetische Nanopartikel, und eines oder beide von
(c) einem Bildgebungsmittel und/oder einem Bildgebungslabel und
(d) mindestens einem Wirkstoff,
wobei der Sphingomyelingehalt der Liposomen im Bereich von 10 mol% bis 45 mol% liegt und
wobei die Liposomen In der Lage sind, ein Bildgebungsmittel und/oder einen Wirkstoff durch die gleichzeitige Wirkung von Sphingomyelinase (SMase) und eines magnetischen Wechselfelds (AMF) in eine Zielumgebung freizusetzen.

2. Zusammensetzung nach Anspruch 1, wobei die magnetischen Nanopartikel einen Kerndurchmesser von 2 nm bis 10 nm aufweisen.

3. Zusammensetzung nach Anspruch 1 oder 2, wobei die magnetischen Nanopartikel ein Metall, eine Metalllegierung, ein Metalloxid, ein Metallhydroxid, ein gemischtes Metalloxid, ein gemischtes Metallhydroxid, ein Metallnitrid, ein Metallsulfid, ein gemischtes Metallnitrid oder ein gemischtes Metallsulfid umfassen, wobei das Metall ausgewählt ist aus der Gruppe bestehend aus Chrom, Mangan, Eisen, Cobalt und Nickel.

4. Zusammensetzung nach einem der Ansprüche 1 bis 3, wobei die Liposomen ferner mindestens ein Bildgebungsmittel, einschließlich radioaktiven Isotopen, Kontrastmittel, Fluorophore und PET-Label, umfassen.

5. Zusammensetzung nach einem der Ansprüche 1 bis 4, wobei der Wirkstoff ein Zytostatikum ist.

6. Zusammensetzung nach einem der Ansprüche 1 bis 5, wobei die Liposomen mindestens ein Therapeutikum, mindestens ein Bildgebungsmittel, und ein Bildgebungslabel umfassen.

7. Zusammensetzung nach einem der Ansprüche 1 bis 6 zur Verwendung in der Therapie und/oder Diagnostik.

8. Zusammensetzung zur Verwendung nach Anspruch 7, wobei die Verwendung in der Therapie die Verwendung der Zusammensetzung zur gezielten Abgabe und Freisetzung eines Wirkstoffs an einem Zielort einschließt und die Verwendung in der Diagnostik die Verwendung der Zusammensetzung als Bildgebungsmittel einschließt.

9. Zusammensetzung nach einem der Ansprüche 1 bis 6 zur Verwendung in der Behandlung von Krebs und/oder zur Verwendung in der Diagnose von Krebs.

10. Zusammensetzung zur Verwendung in einem Verfahren zum Abgeben und Freisetzen eines Wirkstoffs an einem Zielort, umfassend die folgenden Schritte:
(a) Bereitstellen einer Zusammensetzung nach einem der Ansprüche 1 bis 6,
(b) Verabreichen der Zusammensetzung, um die in der Zusammensetzung enthaltenen Liposomen an einem Zielort abzugeben; und
(c) Anwenden von Energie auf die Liposomen und/oder Erhöhen der SMase-Aktivität an dem Zielort, um ein Freisetzen des mindestens einen Wirkstoffs zu bewirken.

11. Zusammensetzung zur Verwendung nach Anspruch 10, wobei der Schritt des Anwendens von Energie auf die Liposomen zu einem Phasenübergang und/oder einer Änderung in der liposomalen Membranstruktur und/oder zu einem Erwärmen der Liposomen führt, um ein Freisetzen des mindestens einen Wirkstoffs zu bewirken.

12. Zusammensetzung zur Verwendung nach Anspruch 10 oder 11, wobei in Schritt (c) die Energie durch Anwenden eines magnetischen Wechselfeldes auf den Zielort angewendet wird.

13. Zusammensetzung zur Verwendung nach einem der Ansprüche 10 bis 12, wobei die SMase-Aktivität durch Verabreichen von exogener SMase oder durch Anwenden von Mitteln zur Induzierung von endogener SMase-Aktivität erhöht wird, um dadurch eine Freisetzung des mindestens einen Wirkstoffs zu bewirken.

14. Zusammensetzung zur Verwendung nach Anspruch 13, wobei die Mittel zum Induzieren der endogenen SMase-Aktivität Strahlung, Aussetzen gegenüber UV-Licht, Anwendung von Wärme und Verabreichung von Chemotherapeutika oder Zellstressinduzierungsmitteln einschließt.

## Revendications

1. Composition comprenant des liposomes, les liposomes comprenant :
(a) des phospholipides, les phospholipides comprenant de la sphingomyéline (SM),
(b) des nanoparticules magnétiques, et un élément ou deux parmi
(c) un agent d'imagerie et/ou un marqueur d'imagerie et
(d) au moins un agent actif,
dans laquelle la teneur en sphingomyéline des liposomes est comprise dans la plage de 10 % en moles à 45 % en moles, et
dans laquelle les liposomes sont en mesure de libérer un agent d'imagerie et/ou un agent actif dans un environnement cible lors de l'action concomitante de sphingomyélinase (SMase) et d'un champ magnétique alternant (AMF).

2. Composition selon la revendication 1, dans laquelle les nanoparticules magnétiques ont un diamètre de noyau compris entre 2 nm et 10 nm.

3. Composition selon la revendication 1 ou 2, dans laquelle les nanoparticules magnétiques comprennent un métal, un alliage métallique, des oxydes métalliques, des hydroxydes métalliques, des oxydes métalliques mélangés, des hydroxydes métalliques mélangés, des nitrures métalliques, des sulfures métalliques, des nitrures métalliques mélangés, ou des sulfures métalliques, dans laquelle le métal est choisi parmi le groupe consistant en le chrome, le manganèse, le fer, le cobalt et le nickel.

4. Composition selon l'une quelconque des revendications 1 à 3, dans laquelle les liposomes comprennent par ailleurs au moins un agent d'imagerie, y compris des isotopes radioactifs, des agents contrastants, des fluorophores et des marqueurs PET.

5. Composition selon l'une quelconque des revendications 1 à 4, dans laquelle l'agent actif est un agent cytostatique.

6. Composition selon l'une quelconque des revendications 1 à 5, dans laquelle les liposomes comprennent au moins un agent thérapeutique, au moins un agent d'imagerie et un marqueur d'imagerie.

7. Composition selon l'une quelconque des revendications 1 à 6 destinée à être utilisée lors d'une thérapie et/ou de diagnostics.

8. Composition à utiliser selon la revendication 7, dans laquelle l'utilisation lors d'une thérapie comporte l'utilisation de la composition en vue de la fourniture et de la libération ciblées d'un agent actif à un site cible, et l'utilisation dans des diagnostics comporte l'utilisation de la composition comme un moyen d'imagerie.

9. Composition selon l'une quelconque des revendications 1 à 6 destinée à être utilisée lors du traitement du cancer et/ou destinée à être utilisée dans des diagnostics du cancer.

10. Composition destinée à être utilisée dans un procédé de fourniture et de libération d'un agent actif sur un site cible comprenant les étapes suivantes :
(a) de mise à disposition d'une composition selon l'une quelconque des revendications 1 à 6,
(b) d'administration de la composition pour mettre à disposition les liposomes contenus dans la composition vers un site cible ; et
(c) d'application d'une énergie sur les liposomes et/ou d'augmentation de l'activité SMase sur l'emplacement cible pour réaliser la libération de l'au moins un agent actif.

11. Composition destinée à être utilisée selon la revendication 10, dans laquelle l'étape de l'application d'une énergie sur les liposomes résulte en une transition de phase et/ou un changement dans la structure de membrane liposomique et/ou un chauffage des liposomes pour réaliser la libération de l'au moins un agent actif.

12. Composition destinée à être utilisée selon la revendication 10 ou 11, dans laquelle lors de l'étape (c), l'énergie est appliquée en appliquant un champ magnétique alternatif sur l'emplacement cible.

13. Composition destinée à être utilisée selon l'une quelconque des revendications 10 à 12, dans laquelle l'activité SMase est augmentée en administrant une SMase exogène ou en appliquant des moyens pour induire une activité SMase endogène pour réaliser la libération de l'au moins un agent actif.

14. Composition destinée à être utilisée selon la revendication 13, dans laquelle les moyens servant à induire une activité SMase endogène comprennent l'irradiation, l'exposition à la lumière UV, l'application de chaleur et l'administration d'agents chimiothérapeutiques ou d'inducteurs de stress de cellule.
